# EUROPÄISCHE PATENTSCHRIFT

(11) **EP 1 516 067 B1**
(45) Veröffentlichungstag und Bekanntmachung des Hinweises auf die Patenterteilung: **10.01.2007**
(21) Anmeldenummer: 03760574.8
(22) Anmeldetag: 22.06.2003
(51) Int. Cl.: C12Q 1/68, B01L 3/00

(54) **DETEKTION VON TOXISCHEN ALGEN**
DETECTION OF TOXIC ALGAE
DETECTION D'ALGUES TOXIQUES

(30) Priorität: 23.06.2002 DE 10228785
(43) Veröffentlichungstag der Anmeldung: 23.03.2005
(73) Patentinhaber: Stiftung Alfred-Wegener-Institut für Polar- und Meeresforschung, 27568 Bremerhaven (DE)
(72) Erfinder: MEDLIN CRAWFORD, Linda, 27619 Schiffdorf-Sellstedt (DE); KERKMANN, Katja, 27628 Sandstedt (DE); LANGE, Martin, 28201 Bremen (DE)
(86) Internationale Anmeldenummer: PCT/DE2003/002124
(87) Internationale Veröffentlichungsnummer: WO 2004/001067

(56) Entgegenhaltungen:
- DE-A- 10 032 042
- US-A- 5 582 983
- US-A- 5 958 689
- MANHART JAMES R ET AL: "Pseudo-nitzschia pungens and P. multiseries (Bacillariophyceae): Nuclear ribosomal DNAs and species differences" JOURNAL OF PHYCOLOGY, Bd. 31, Nr. 3, 1995, Seiten 421-427, XP008026323 ISSN: 0022-3646
- UWE JOHN: "Biology of toxic algae : a study of species of the genus Chrysochromulina (Prymnesiophyceae) and Alexandrium (Dinophyceae)" April 2002 (2002-04), UNIVERSITÄT BREMEN , BREMEN , XP002352491 Gefunden im Internet: URL:http://elib.suub.uni-bremen.de/publica tions/dissertations/E-Diss525_john.pdf> * Seite 160 * * Seite 180 * * Seite 198 * * Seiten 208-209 * * Seiten 218-219 *
- MILLER PETER E ET AL: "Identification and enumeration of cultured and wild Pseudo-nitzschia (Bacillariophyceae) using species-specific LSU rRNA-targeted fluorescent probes and filter-based whole cell hybridization" JOURNAL OF PHYCOLOGY, Bd. 34, Nr. 2, April 1998 (1998-04), Seiten 371-382, XP008026094 ISSN: 0022-3646
- CAMPBELL CHARLES N ET AL: "Enzyme-amplified amperometric sandwich test for RNA and DNA." ANALYTICAL CHEMISTRY. UNITED STATES 1 JAN 2002, Bd. 74, Nr. 1, 1. Januar 2002 (2002-01-01), Seiten 158-162, XP002266642 ISSN: 0003-2700
- GROBEN RENE ET AL: "rRNA probes for identification and characterization of marine phytoplankton: Their potential application for DNA microchips" PHYCOLOGIA, Bd. 40, Nr. 4 Supplement, Juli 2001 (2001-07), Seite 123, XP008026020 7th International Phycological Congress;Thessaloniki, Greece; August 18-25, 2001 ISSN: 0031-8884
- GROBEN RENE ET AL: "AIMS: An Automated Identification System For Microbial Populations." PROCEEDINGS OF THE 15TH INTERNATIONAL DIATOM SYMPOSIUM, 2002, Seiten 21-29, XP001156033 15th International Diatom Symposium;Perth, WA, Australia; September 28-October 06, 1998, A.R.G. Gantner Verlag KG, FL-9491, P. O. Box 131, Ruggell, Liechtenstein Series: Proceedings of the International Diatom Symposium (ISSN 0933-0755 (ISSN print)) ISBN: 3-904144-81-2 (cloth)
- DATABASE EMBL [Online] 8. November 1995 (1995-11-08), "Alexandrium ostenfeldii 18S small subunit ribosomal RNA." XP002352495 gefunden im EBI accession no. EM_PRO:AO27500 Database accession no. AO27500
- DATABASE EMBL [Online] 11. Oktober 1997 (1997-10-11), "Alexandrium tamarense 18S ribosomal RNA gene, complete sequence." XP002352494 gefunden im EBI accession no. EM_PRO:AF022191 Database accession no. AF022191
- SIMON NATHALIE ET AL: "Oligonucleotide probes for the identification of three algal groups by dot blot and fluorescent whole-cell hybridization" JOURNAL OF EUKARYOTIC MICROBIOLOGY, Bd. 47, Nr. 1, Januar 2000 (2000-01), Seiten 76-84, XP008026018 ISSN: 1066-5234
- FUCHS BERNHARD M ET AL: "Unlabeled helper oligonucleotides increase the in situ accessibility to 16S rRNA of fluorescently labeled oligonucleotide probes" APPLIED AND ENVIRONMENTAL MICROBIOLOGY, Bd. 66, Nr. 8, August 2000 (2000-08), Seiten 3603-3607, XP002266643 ISSN: 0099-2240
- SCHOLIN CHRISTOPHER A ET AL: "DNA probes and a receptor-binding assay for detection of Pseudo-nitzschia (Bacillariophyceae) species and domoic acid activity in cultured and natural samples" JOURNAL OF PHYCOLOGY, Bd. 35, Nr. 6 Suppl., 1999, Seiten 1356-1367, XP002266644 ISSN: 0022-3646
- WALSH DAVID ET AL: "Heterogeneity of SSU and LSU rDNA sequences of Alexandrium species" BIOCHEMICAL SYSTEMATICS AND ECOLOGY, Bd. 26, Nr. 5, Juli 1998 (1998-07), Seiten 495-509, XP002266645 ISSN: 0305-1978
- 'Internationaler Code der Botanischen Nomenklatur' WIKIPEDIA, [Online] Gefunden im Internet: <URL:http://de.wikipedia.org/wiki/Internati onaler_Code_der_Botanischen_Nomenklatur>
- METFIES K.; HULJIC S.; LANGE M.; MEDLIN L.K.: 'Electrochemical detection of the toxic dinoflagellate Alexandrium ostenfeldii with a DNA-biosensor' BIOSENSORS AND BIOELECTRONICS Bd. 20, 07 Juli 2004, Seiten 1349 - 1357
- 'Taxon: Genus Pseudonitzschia' THE TAXONOMICON, [Online] Gefunden im Internet: <URL:http://sn2000.taxonomy.nl/Taxonomicon/ TaxonTree.aspx?id=156736>
- SIMS ET AL: 'Evolution of the diatoms: insights from fossil, biological and molecular data' PHYCOLOGIA Bd. 45, Nr. 4, 2006,

### Sequenzprotokoll

<110> Stiftung Alfred-Wegener-Institut für Polar- und Meeresforschung, Columbusstrasse, 27568 Bremerhaven, Deutschland
<120> Detektion von toxischen Algen
<130> AWI 01/0602DE
<160> 20
<210> 1
   <211> 18
   <212> DNA
   <213> Heterokonta, toxisch
<400> 1
   acgacttcac cttcctct 18
<210> 2
   <211> 18
   <212> DNA
   <213> Prymnesium gl01A, toxisch
<400> 2
   tgctcgccaa cgaggtgt 18
<210> 3
   <211> 18
   <212> DNA
   <213> Prymnesium gl02B, toxisch
<400> 3
   aagaagtgct cgccaacg 18
<210> 4
   <211> 18
   <212> DNA
   <213> Pseudo-nitzschia
<400> 4
   cagtacagcg caatcact 18
<210> 5
   <211> 18
   <212> DNA
   <213> Alexandrium tamarense/fundyense/catenella species complex, toxisch
<400> 5
   ttcaaggcca aacacctg 18
<210> 6
   <211> 18
   <212> DNA
   <213> Alexandrium tamarense (North America)
<400> 6
   aacactccca ccaagcaa 18
<210> 7
   <211> 18
   <212> DNA
   <213> Gymnodinium mikimotoi species complex
<400> 7
   ttccgggcaa ggtcgaaa 18
<210> 8
   <211> 18
   <212> DNA
   <213> Prorocentrum lima
<400> 8
   ccttctgcta agtcgggt 18
<210> 9
   <211> 18
   <212> DNA
   <213> Prorocentrum lima
<400> 9
   ctctagcatt tccacggg 18
<210> 10
   <211> 18
   <212> DNA
   <213> Prorocentrum lima species
<400> 10
   acaccccaat tgcctcgt 18
<210> 11
   <211> 18
   <212> DNA
   <213> Prorocentrum lima species cluster
<400> 11
   gcaatcagaa cccatcct 18
<210> 12
   <211> 18
   <212> DNA
   <213> Alexandrium ostenfeldii (G+15)
<400> 12
   caccaaggtt ccaagcag 18
<210> 13
   <211> 18
   <212> DNA
   <213> Alexandrium ostenfeldii (D)
<400> 13
   tccatgtagc aatcgacc 18
<210> 14
   <211> 17
   <212> DNA
   <213> Pseudo-nitzschia australis
<400> 14
   aacgtcgttc cgccaat 17
<210> 15
   <211> 18
   <212> DNA
   <213> Pseudo-nitzschia multiseries
<400> 15
   cgccgccaaa aggcatgc 18
<210> 16
   <211> 18
   <212> DNA
   <213> Pseudo-nitzschia pungens
<400> 16
   gggcaccctc agtacgac 18
<210> 17
   <211> 18
   <212> DNA
   <213> Alexandrium minutum
<400> 17
   tccaggcaag gttgcaaa 18
<210> 18
   <211> 23
   <212> DNA
   <213> Alexandrium tamarense
<400> 18
   tgcacctctg ttggtrrtac att (R=A/G) 23
<210> 19
   <211 > 22
   <212> DNA
   <213> Alexandrium ostenfeldii (Fänger)
<400> 19
   caacccttcc caatagtcag gt 22

## Beschreibung

Die Erfindung bezieht sich im Zusammenhang mit der Detektion von toxischen Algen auf sequenzspezifische Fängersonden zur Algendetektion durch selektive, unter Hybridisierungsbedingungen erfolgende Hybridisierung einer komplementären, die zu detektierende Alge eindeutig charakterisierenden Nukleinsäuresequenz. Ein weiterer Gegenstand der Erfindung ist ein elektrochemisches Nachweisverfahren zur schnellen Vor-Ort-Detektion von toxischen Algen in einer flüssigen Probe unter Verwendung zumindest einer immobilisierten, sequenzspezifischen Fängersonde. Weitere Gegenstände der Erfindung sind eine Anordnung zur Durchführung des Nachweisverfahrens und eine Verwendung der Anordnung.

Das Vorkommen von toxinbildenden Kontaminierungsspezies in marinen Ökosystemen ist seit Langem bekannt. Nach Kontakt mit solchen Organismen oder Verzehr von mit Toxinen angereicherten Meeresorganismen können physiologische Schäden beim Menschen auftreten, die schlimmstenfalls bis zum Tode führen können. Gerade in den letzten zehn bis zwanzig Jahren werden vermehrt toxische Algenblüten beobachtet, die ernste ökologische und gesundheitliche Probleme zur Folge haben können. Marine Ökosysteme können gestört werden, massives Algenwachstum und Algengiftsyndrome können auftreten. Auch ökonomische Folgen ergeben sich für die betroffenen Gegenden. Das örtliche Auftreten von toxischen Algen erfolgt jedoch oft äußerst plötzlich und nur vorübergehend, sodass Untersuchungen erschwert werden. In den letzten Jahren wurden eine Reihe neuer bioaktiver, toxischer Substanzen isoliert. Eine qualitative und quantitative Analysemöglichkeit der unterschiedlichen marinen Verursacher für die Produktion von Toxinen ist deshalb dringend erforderlich. Konventionelle Untersuchungsmethoden sind jedoch zeitaufwändig und schwierig, sodass sie nur in Testlaboren von Fachpersonal durchgeführt werden können. In der **Veröffentlichung I** "Unbekanntem Leben auf der Spur" von R. Amman et al., Naturwissenschaftliche Rundschau 45, Heft 9/92, 348-351, werden DNA-Sonden zur Identifizierung von nichtkultivierbaren Bakterien beschrieben. Eine Fängersonde in Form einer einzelsträngigen DNA-Sequenz bindet unter genau definierten Hybridisierungsbedingungen sequenzspezifisch an eine komplementäre DNA- oder RNA-Sequenz. Der Hybridisierung zweier Nukleinsäuremoleküle liegt das Prinzip der spezifischen Basenpaarung von Adenin (A) und Thymin (T) (Uracil (U) im Fall von RNA) und von Guanin (G) mit Cytosin (C) zugrunde. Aktuell gewinnen daher verfügbare molekulare Nukleinsäure-Sonden, die bestimmte Zielsequenzen, insbesondere ribosomale RNA erkennen, Antikörper, die bestimmte Zelloberflächen-Moleküle bzw. cytoplasmatische Biomoleküle binden, und andere molekulare und biochemische Verfahren für die Detektion von Kontaminierungsspezies an Bedeutung. Dazu zählen vor allem auch molekulargenetische Untersuchungen und hierauf aufbauend die Etablierung einer auf eindeutig speziescharakterisierenden DNA-Sequenzen basierenden Taxonomie, die das Erkennen potenziell toxischer Mikroalgen und Bakterien ermöglicht. Als Ergebnis dieser Untersuchungen lassen sich spezies-, gattungs- und klassenspezifische DNA-Sequenzen definieren, die als hochspezifische DNA-Sonden zur eindeutigen Identifizierung der jeweiligen Mikroorganismen verwendet werden können.

Der Wert einer DNA-Sonde besteht also in der eindeutigen Identifikation eines Organismus oder einer Organismengruppe. Jeder eukaryontische Organismus besitzt eine kodierende Gensequenz für die kleine Untereinheit 18S und die große Untereinheit 28S im Ribosom. Diese werden für die Identifikation von Organismen benutzt, da ihre Sequenz für jeden Organismus hochspezifisch ist. Dabei reicht es jedoch nicht aus, ein beliebig langes Fragment aus der 18S-oder 28S-DNA auszuwählen, es muss vielmehr ein spezieller Sequenzabschnitt gefunden werden, der den geforderten Kriterien wie Spezifität für eine Klasse, Gattung oder Spezies eindeutig entspricht. Für die Ermittlung von molekularen Sonden, die als spezifisch betrachtetet werden können, ist daher eine langwierige und mühsame Entwicklungsarbeit erforderlich. Aus der **US 5.582.983** (Anderson, Scholin) sind sequenzspezifische Fängersonden zur Identifikation von einigen Alexandrium-Spezies (dinophyceae) bekannt. In der **US 5.958.689** (Scholin, Haydock, Cangelosi) werden sequenzspezifische Fängersonden für einige toxische marine- Kieselalgen der Gattung Pseudo-Nitzschia offenbart. Aufgrund der Artenvielfalt mariner Algen ist die durch die beiden genannten Druckschriften und durch weitere, über die GenBank NCBI (National Center for Biotechnology Information, http://www.ncbi.nlm.nih.gov, Stand 23./26.01.2001) gegebene Sondenliste für marine Algen jedoch bei Weitem nicht vollständig. Weitere Sonden zur Detektion toxischer Algen werden in der Veröffentlichung von Simon, N. et al.: "Oligonucleotide probes for the identification of three algal groups by dot blot and fluorescent whole-cell hybridization" JOURNAL OF EUKARYOTIC MICROBIOLOGY, Bd. 47, Nr. 1, Januar 2000, S.76-84 beschrieben. Mit der vorliegenden Erfindung werden einige neue sequenzspezifische Fängersonden zur Detektion von Algen genannt, die sich durch ihre Toxizität auszeichnen. Durch ein mögliches epidemisches Auftreten sind diese toxischen Algen im Zusammenhang mit der bereits weiter oben angesprochenen Problematik für Küstenländer von besonderem Interesse. Dabei arbeiten die Fängersonden mittels selektiver, unter Hybridisierungsbedingungen erfolgender Hybridisierung einer komplementären, die zu detektierende toxische Alge eindeutig charakterisierenden Nukleinsäuresequenz aus der rRNA der kleinen Untereinheit 18S oder der großen Untereinheit 28S des algenspezifischen Ribosoms. Die Charakterisierungen der einzelnen Fängersonden sind dem beigefügten Sequenzprotokoll zu entnehmen.

Um ein als Sonde verwendetes Nukleinsäure-Molekül nach der Hybridisierung mit einer komplementären DNA oder RNA zu detektieren, werden Nukleinsäure-Sonden mittels eines Reportermoleküls markiert. Beispielsweise können als Sonde fluoreszenzmarkierte Oligonukleotide eingesetzt werden, um spezifisch ribosomale RNA (rRNA) einer Spezies zu detektieren. Aus einem beliebigen Biotop, beispielsweise aus Meerwasserproben, kann somit gezielt eine Spezies aufgrund ihrer spezifischen rRNA-Sequenz detektiert werden. Aus der **US 5.344.757** ist der Nachweis von Nukleinsäuren nach deren Hybridisierung mit einer markierten DNA-Sonde bekannt (Sandwichhybridisierung). Dabei ist die zum Einsatz kommende DNA-Sonde mit einem Reportermolekül, einem z.B. Steroid-Hapten, wie Digoxigenin, kovalent verknüpft, das wiederum mit Hilfe eines markierten Anti-Rezeptormolekül-Antikörpers detektiert werden kann. Die Detektion von Nukleinsäuren nach einer Hybridisierung mit einer Enzym-markierten DNA-Sonde wird in der **US-5.867.928** beschrieben, wobei das Enzym β-Galaktosidase entweder direkt, über eine SH-Gruppe, oder nach Modifikation mit Biotin oder Avidin an die DNA-Sonde gekoppelt vorliegt und das aus der Reaktion mit β-Galaktosidase entstandene Produkt - als Substrat wird o-nitrophenyl-β-D-galactopyranosid eingesetzt - UV-optisch detektiert wird.

Aus der **DE 100-32 042 A1** ist ein elektrochemischer Einwegbiosensor für die quantitative Bestimmung von Analytkorizentrationen in Flüssigkeiten bekannt. Bei dem zugrundeliegenden Detektionsverfahren handelt es sich um ein elektrochemisches Nachweisverfahren unter Verwendung einer immobilisierten, sequenzspezifischen Fängersonde zur selektiven, unter Hybridisierungsbedingungen erfolgenden Hybridisierung einer komplementären Nukleinsäuresequenz des nachzuweisenden Analyts. Gemäß Beispiel 4 der genannten Druckschrift erfolgt eine Detektion jedoch durch einfache Hybridisierung, indem der nachzuweisende DNA-Strang direkt mit Digoxigenin markiert wird. Nach erfolgter Hybridisierung des komplementären Einzelstranges an den immobilisierten Einzelstrang als Fängersonde erfolgt der Nachweis nach Zugabe eines Enzymkonjugates, das aus einem Antikörper und einem Enzym, das die Digoxigenin-Markierung erkennt, besteht. Durch Oxidation und Reduktion eines umgebenden Mediators erfolgt anschließend unter Anlegen einer vorgegebenen Messspannung eine amperometrische oder zyklovoltametrische Auswertung. Aufgrund der direkten Markierung der nachzuweisenden DNA-Sequenz ist das bekannte elektrochemische Nachweisverfahren allerdings relativ aufwändig durchzuführen. Es verfügt außerdem nur über eine relativ geringe Nachweisempfindlichkeit. Als Aufgabe für das erfindungsgemäße Nachweisverfahren ist daher eine deutliche Effizienzsteigerung bei der Nachweismöglichkeit von toxischen Algen bei einer gleichzeitigen Verfahrensvereinfachung anzusehen.

Als Lösung hierfür ist das beanspruchte elektrochemische Nachweisverfahren gemäß **Anspruch 1** vorgesehen, das ausgehend von dem gemäß der **DE 100 32 042 A1** bekannten elektrochemischen Nachweisverfahren zwei essenzielle Verfahrensweiterbildungen umfasst. Zum einen erfolgt bei dem erfindungsgemäßen Nachweisverfahren eine Sandwichhybridisierung unter Beteiligung einer Detektorsonde. Dadurch entfällt die direkte Markierung der zu detektierenden DNA oder RNA der nachzuweisenden Algenspezies. Die Markierung, beispielsweise mit einem Antigen, wird in einfacher und allgemein vorbereitender Weise an der Detektorsonde angebracht. Das grundlegende Prinzip der Sandwichhybridisierung ist beispielsweise im Zusammenhang mit einem Nachweisverfahren durch Enzymamplifikation der Veröffentlichung II von B. Wicks et al.: "A Sandwich Hybridization Assay Employing Enzyme Amplification for Determination of Specific Ribosomal RNA from Unpurified Cell Lysates" (Analytical Biochemistry 259 (1998), pp 258-264) zu entnehmen. Es bietet den großen Vorteil gegenüber der einfachen Hybridisierung, dass auch ungereinigte Proben analysiert werden können.

Des Weiteren wird bei dem erfindungsgemäßen Verfahren eine dritte Sonde in Form einer Helfersonde verwendet. Diese ermöglicht bei einigen Speziesarten, beispielsweise Alexandrium tamarense, überhaupt erst den Nachweis. Bei anderen Algenarten erhöht der Einsatz einer Helfersonde die Nachweisgrenze bedeutend. Die Funktion einer Helfersonde, die darin besteht, dass durch Anlagerung an die zu detektierende DNA oder RNA Faltungen des Stranges vermieden werden, ist allgemein beispielsweise aus der **Veröffentlichung III** von B. M. Fuchs :"Unlabeled Helper Oligonucleotides Increase the In Situ Accessibility to 16S rRNA of fluorescently Labeled Oligonucleotide Probes" (Applied and Environmental Microbiology, Aug. 2000, pp 3603-3607) bekannt. Die gleichzeitige Einführung sowohl der Sandwichhybridisierung unter Verwendung einer Detektorsonde als auch die Verwendung einer zusätzlichen Helfersonde bei dem erfindungsgemäßen Nachweisverfahren erbringt die gewünschte deutliche Effizienzsteigerung gegenüber dem bekannten elektrochemischen Nachweisverfahren.

Ausschlaggebend für den effektiven Einsatz von Detektor- und Helfersonden ist deren molekularer Aufbau. Auch diese Sonden sind ebenfalls spezifisch, aber nicht hochspezifisch und damit speziescharakterisierend wie die Fängersonden. Sie binden daher nicht an die die zu detektierende Alge eindeutig charakterisierende Nukleinsäuresequenz, sondern an relativ eng dazu benachbarte Sequenzen in der rRNA (oder der einzelsträngigen DNA, sofern diese vorhanden ist). Zur Herstellung geeigneter Detektor- und Helfersonden ist ein maximal erlaubter Abstand der beiden zusätzlichen Sonden vom Ankopplungsort der Fängersonde-zu definieren. Gute Ergebnisse erhält man, wenn dieser Sequenzabstand möglichst gering mit einer maximalen Differenz von 150 bis 200 Basen festgelegt wird. Bei Kenntnis der algencharakterisierenden Fängersonden, wie sie bei dem erfindungsgemäßen Nachweisverfahren gemäß **Anspruch 2** verwendet werden können, in der kleinen oder großen Untereinheit des Ribosoms ist somit aufgrund der Kenntnis der gesamten Sequenz zumindest in diesem Bereich die Definition entsprechend geeigneter Detektor- und Helfersonden für die einzelnen Algenspezies ohne Weiteres möglich. Die Anlagerung der zusätzlichen Sonden an die zu detektierenden Alge erfolgt dann unter den gleichen Hybridisierungsbedingungen wie bei der Anlagerung der charakteristischen Nukleinsäuresequenz an die Fängersonde.

Zur Durchführung des aus der **DE 100 32 042 A1** bekannten elektrochemischen Nachweisverfahrens werden ebendort preiswerte Einwegbiosensorchips und ein anzeigendes Handgerät mit einer Potentiostateinheit beschrieben, in das die Sensorchips zur Durchführung der Messung eingeschoben werden. Der kommerziell erhältliche Einwegbiosensorchip ist mit einer Dreielektroden-Anordnung ausgestattet (Arbeits-, Referenz- und Hilfselektrode). Er wird zur leichten Handhabung über einen Kontaktstreifen in das Handgerät eingeschoben. Die Arbeitselektrode des Chiprohlings wird je nach zu detektierender Substanz in unterschiedlicher Weise modifiziert. Um die Konzentration eines Analyten zu bestimmen, wird dieser auf die Arbeitselektrode pipettiert. Die Messung erfolgt amperometrisch oder zyklovoltametrisch mit Hilfe der in das Handgerät integrierten Potentiostateinheit, über die Messspannungen vorgebbar sind. Die Konzentration der Analysenprobe kann nach sehr kurzer Messzeit auf dem Display des Handgerätes, das gerade auch von ungeschulten Anwendern problemlos bedient werden kann, abgelesen werden. Einwegbiosensorchips und ein Handgerät mit einer Potentiostateinheit sind ebenfalls Bestandteile der beanspruchten Anordnung zur Durchführung des elektrochemischen Verfahrens. Mit dieser Anordnung wird ein komplettes Set, das neben den beiden genannten Hauptkomponenten und den erforderlichen Reagenzien nur noch einen oder mehrere Behälter zur Aufbereitung der zu untersuchenden flüssigen Probe enthält, auch dem unkundigen Anwender zur Verfügung gestellt, mit er problemlos das Nachweisverfahren für toxische Algen vor Ort durchführen kann.

Dabei kann das Set einen oder mehrere Einwegbiosensoren mit sequenzspezifischen Fängersonden in Form von Basensequenzen enthalten. Möglich ist auch eine Kombination von mehreren verschiedenen Fängersonden auf einem Einwegbiosensorchip, um durch die Verwendung nur eines Sensorchips zumindest eine qualitative Aussage über das Vorhandensein eines Spektrums toxischer Algen in der zu untersuchenden Probe zu erhalten. Derzeit ist der Nachweis von toxischen Algen mittels Nukleinsäure-Sonden (vgl. **US 5.582.983** und **US 5.958.689**) relativ zeitaufwändig und erfordert spezielles Fachwissen. Er kann nur in Testlabors von geschultem Personal durchgeführt werden. Bei dem Nachweisverfahren nach der Erfindung beträgt der Zeitaufwand unter Einsatz vorkonfektionierter Einwegbiosensoren mit immobilisierten Fängersonden sowie passend vorrätig gehaltenen Detektor- und Helfersonden nur mehr ungefähr drei Stunden und kann auch von ungeschultem Personal durchgeführt werden.

In der **Veröffentlichung IV** von M.I**.** Pividori et al.: "Dot-blot amperometric genosensor for detecting an novel determinant of β-lactamase resistance in Staphylcoccus aureus" (aus Analyst, 2001, 126, pp 1551-1557) wird ebenfalls ein elektrochemisches Nachweisverfahren beschrieben, hier aber mit einem grundlegend zu dem bekannten Einwegbiosensor für das Handgerät unterschiedlichen Sensoraufbau. Es wird eine Dot-Blot-Hybridisierung auf einer Membran durchgeführt, die dann für die Detektion auf den Sensor gespannt wird. Es erfolgt also eine indirekte, einfache Hybridisierung. Die algenspezifische Nukleinsäuresequenz der nachzuweisenden Alge wird nach einer zunächst erforderlichen Amplifikation durch polymerase Kettenreaktion (PCR) auf der Membran immobilisiert und im Anschluss mittels einer biotinylierten Fängersonde nachgewiesen. Des Weiteren wird in der Veröffentlichung über eine doppelte Hybridisierung mit untersehiedtichen Sonden berichtet.

Eine besonders bevorzugte Anwendung der mit vorliegender Erfindung Beschriebenen Anordnung ist in der Verwendung als Frühwarnsystem vor toxischen Algen zu sehen. Dadurch wird mit der vorliegenden Erfindung ein effektives Frühwarnsystem zur Analyse von Phytoplankton mit dem Schwerpunkt auf toxischen Meeresmikroorganismen (Mikroalgen und Bakterien) zur Verfügung gestellt, wie es bislang nicht existiert hat. Es ist wartungs- und kostengünstig aufgebaut, äußerst einfach zu handhaben und leicht zu transportieren, sodass es eine höchste Einsatzflexibilität bezüglich des Messortes für individuelle, aber auch routinemäßige Messungen erreicht. Durch die Integration der Probenaufbereitung und Hybridisierung ergibt sich ein umfassendes System, bei dem Zusatzelemente oder Aufbereitungsschritte in Fremdlaboren vollständig entfallen. Weiterhin ist das Frühwarnsystem durch die eingesetzte Technologie in Bezug auf die verwendeten Biosensoren mit selektiven Sonden in Form von einsteckbaren Einwegchips leicht zu handhaben und damit auch dem ungeschulten Nutzer ohne Weiteres zugänglich. Mit den speziellen Sonden können gefährliche Meeresmikroorganismen direkt vor Ort auch in geringer Zellzahl mit hoher Sensitivität zuverlässig und schnell aufgespürt werden. Routinemäßige Überwachungen sind somit mit einem minimalen Geräteaufwand möglich. Bei der beanspruchten Anordnung in der Anwendung als Frühwarnsystem vor toxischen Algen handelt es sich damit um ein marktfähiges Produkt, mit dem schnell und zuverlässig hochsensitive Ergebnisse erzielt werden. Dadurch kann der Populationsdynamik der toxischen Algen in Echtzeit gefolgt werden.

Die Anordnung nach der Erfindung in der beanspruchten Anwendung als Frühwarnsystem vor toxischen Algen kann allgemein bei verschiedenen Forschungseinrichtungen für wissenschaftliche Untersuchungen (Biodiversitätsforschung) eingesetzt werden, es kann aber auch als Frühwarnsystem äußerst wirkungsvoll dabei helfen, beispielsweise das Wuchsverhalten von toxischen Algenblüten in Echtzeit zu ermitteln, bevor diese die Küsten bevölkern und Störungen oder sogar Schaden für die Menschen bewirken können. Das System kann beispielsweise von Kommunen und Behörden auf den Gebieten des Tourismus, des Umweltschutzes oder der Fischerei wirkungsvoll genutzt werden. Gerade durch gesetzliche Regelungen auferlegte Überwachungspflichten an Küsten und Gewässern können durch einen Einsatz der Anordnung nach der Erfindung in ihrer Umsetzung deutlich vereinfacht und verbessert werden. Dazu wird eine Meerwasserprobe nach der entsprechenden Aufbereitung (unter Umständen durch Hinzuziehung von Hilfe durch Dritte) einfach auf einen Einwegbiosensorchip, der eine der für die Gegend charakteristische, toxische Algenbelastung entsprechende Sondensequenz aufweist aufgetropft. Nach erfolgter Sandwichhybridisierung wird der Analysedurchlauf durch Einschieben des betropften Einwegbiosensorchips gestartet. In kürzester Zeit liefert das Gerät ohne weitere Hilfe von Außen, die zusätzlich Zeit und Weg kostet, selbst bei geringen Spurenvorkommen einen sicheren qualitativen und quantitativen Warnhinweis. So können toxische Algenblüten, wie sie in den letzten Jahren vor allem in Küstenregionen aufgetreten sind, frühzeitig erkannt werden. Die Verhinderung derartiger massiver Verseuchungen, die innerhalb kürzester Zeit eintreten können, sind gerade auch für Gebiete, die vom Tourismus und/oder der Fischereiwirtschaft leben, von existenzieller Bedeutung.

Im Folgenden soll die Erfindung in ihrem Umfang, ihren Grundlagen und einzelnen Ausprägungsformen anhand von Ausführungsbeispielen und einer **Figur,** die in schematischer Darstellung den Nachweis der Sandwichhybridisierung auf einem Einwegbiosensorchip zeigt, näher erläutert werden.

In der **Figur** ist im linken Figurenteil eine vergrößerte Detailzeichnung eines Einwegbiosensorchips **1** dargestellt. Auf einem Chipträger **2** befindet sich der Sensor **3** mit einer Arbeitselektrode **4,** einer Referenzelektrode **5** und einer Hilfselektrode **6.** Der Sensor **3** arbeitet amperometrisch: zwischen der Arbeitselektröde **4** mit der immobilisierten, sequenzspezifischen Fängersonde (einzelsträngige DNA, vgl. rechten Figurenteil) und der Referenzelektrode **5** wird eine vorgegebene Messspannung angelegt; gemessen wird dann der Strom, der zwischen der Arbeitselektrode **4** und der Hilfselektrode **6** in der Chipbeschichtung fließt, wobei der resultierende Strom direkt proportional zur auftretenden Konzentration eines enzymatischen Produktes und somit direkt proportional zur Konzentration der gesuchten speziellen toxischen Alge ist. Im nachfolgend ausgeführten Ausführungsbeispiel resultiert der gemessene Strom aus der Oxidation des aus der Enzymreaktion entstandenem Produktes p-Aminophenol zu p-Iminochinon.

Im rechten Figurenteil der **Figur** ist die Arbeitselektrode **4** nochmals vergrößert dargestellt, um die Sandwichhybridisierung für den Detektionsvorgang besser darstellen zu können. Die molekulare, sequenzspezifische Fängersonde **7** ist über Biotin **8** an Avidin **9** gebunden. Das Avidin **9** ist auf die Oberfläche des Chipträgers **2** im Bereich der Arbeitselektrode **4** aufgebracht, sodass über die Verbindung die Fängersonde **7** auf der Chipoberfläche immobilisiert ist. Die algencharakteristische Nukleinsäuresequenz **10** (RNA oder einzelsträngige DNA) der nachzuweisenden Alge wurde in einem vorangegangenen Hybridisierungsschritt mit einer niederspezifischen Detektorsonde **11** und einer niederspezifischen Helfersonde **12** versehen. Dabei liegt die Helfersonde **12** näher an der algencharakteristischen. Nukleinsäuresequenz **10,** um deren Bindung zu erleichtern, indem die Bildung von Sekundärstrukturen in diesem Bereich durch die Präsenz der Helfersonde **12** erschwert werden. Die Detektorsonde **11** ist mit einem Antigen **13** dig-markiert. Nach Aufpipettierung und Hybridisierung der algencharakteristischen Nukleinsäuresequenz **10** an die sequenzspezifische Fängersonde **7** liegt eine Sandwichhybridisierung vor. Dabei wirkt die Helfersonde **12** hybridisierungsermöglichend (beispielsweise bei der Neurotoxin bildenden Alge *Alexandrium tamarense*) oder zumindest -verstärkend. Zur amperometrischen Detektion wird ein Enzymkonjugat (Anti-Dig-Antikörper-POD-Konjugat) auf die Arbeitselektrode **4** pipettiert, wobei die mit dem Enzym **14** markierten Antikörper **15** an die Antigene **13** der Detektorsonden **11** ankoppeln. Durch Reduktion/Oxidation (vgl. weiter unten) tritt ein Stromfluss in einem umgebenden Mediator auf, der proportional zur Konzentration der an die Fängersonden **7** hybridisierten algencharakteristischen, sequenzspezifischen Nukleinsäuresequenzen **10** und damit das Maß für die nachzuweisende toxische Alge ist.

Im vorhergehenden Ausführungsbeispiel wird die Immobilisierung einer Fängersonde, das heißt einer Fängersondenart auf dem Einwegbiosensorchip beschrieben. Mit einer Vielzahl von immobilisierten Fängersonden mit spezieller Basensequenz ist somit eine toxische Alge bzw. eine Spezies mit enger Artverwandtheit zu detektieren. Dabei stiegt die Empfindlichkeit des Nachweisverfahrens mit der Anzahl der immobilisierten Fängersonden auf einem Sensorchip. Somit sind zum Nachweis unterschiedlicher toxischer Algen entsprechend verschiedene Sensorchips zu verwenden. Möglich ist auch die Immobilisierung verschiedener Fängersonden für unterschiedliche toxische Algen auf einem gemeinsamen Sensorchip, um in einem Verfahrensdurchgang bereits ein ganzes Spektrum toxischer Algen nachweisen zu können.

Zu dem Nachweisverfahren von toxischen Algen gemäß der vorliegenden Erfindung gehören in der Hauptsache die folgenden Verfahrensschritte :

### • Probenaufbereitung

Die Isolation der gesamt-RNA aus der auf Vorhandensein von toxischen Algen zu untersuchenden flüssigen Probe, beispielsweise Meerwasser, erfolgt mittels eines fertig vorbereiteten Kits in einem Aufbereitungsbehälter (z. B. Firma Qiagen (Hilden)).

### • Vorbereitung der Einwegbiosensorchips für die Sandwichhybridisierung

Die Arbeitselektrode auf einem Einwegbiosensorchip als Chiprohling wird zunächst mit Avidin beschichtet. Danach erfolgt die Anbindung der Fängersonde (Fängeroligonukleotid) über Biotin an die avidinbeschichtete Elektrode (vgl. Figur). Dabei wird ausgenutzt, dass Avidin eine hohe Bindungsaffinität für Biotin aufweist. Die so vorbereiteten Einwegbiosensorchips sind bei 4°C mindestens drei Monate haltbar.

### • Hybridisierung der Gesamt-RNA aus der aufbereiteten Probe an den vorbereiteten Einwegbiosensorchip

Im Hybridisierungsansatz ist gesamt-RNA, die Helfersonde (Helferoligonukleotid) und die Detektorsonde (Detektoroligonukleotid) enthalten. Die Hybridisierung der beiden Sonden an die nachzuweisende Alge erfolgt im Hybridisierungspuffer. Die Hybridisierung der mit der Helfer- und der Detektorsonde vorbereiteten algenspezifischen Nukleinsäuresequenz an die auf dem Einwegbiosensorchip immobilisierte Fängersonde erfolgt auf dem Einwegbiosensorchip, nachdem der Hybridisierungsansatz auf den Sensorchip pipettiert wurde. Es liegt dann eine Sandwichhybridisierung zwischen Fänger- und Detektorsonde unter Zuhilfenahme einer Helfersonde vor, die die Bindung des Fängeroligonukleotids an die Zielsequenz erleichtern soll.

### • Detektion der Sandwichhybridisierung

Die Detektion der Sandwichhybridisierung erfolgt über eine Enzymreaktion, die ebenfalls auf dem Sensor stattfindet. Das Enzym ist an einen Antikörper (beispielsweise von Firma Roche Diagnostics GmbH in Mannheim) gekoppelt, der ein Epitop (spezielle Antigenbereiche, die spezifisch von Antikörpern erkannt werden) erkennt, das an die Detektorsonde gekoppelt ist (vgl. Figur). Im Anschluss an die Antikörperreaktion erfolgt die Detektion der Sandwichhybridisierung über die Enzymreaktion nach Zugabe einer Substratmischung, die den Mediator und H₂O₂ enthält. Der hybridisierte Einwegbiosensorchip wird dann durch Einschieben in das Handgerät kontaktiert. Vom Handgerät wird der Elektronenfluss während einer Oxidationsreaktion gemessen. Die Substrate der Reaktion sind H₂O₂ (Wasserstoffperoxid als Oxidationsmittel) und N-Phenyl-1,4-phenylenediamin-hydrochlorid (Mediator). Die Reaktion von Wasserstoffperoxid zu Wasser wird durch das Enzym "Horse-radish peroxidase (POD)" katalysiert, das an einen Antikörper gekoppelt ist, der die an eine Dig-Markierung (Digoxigenin) gekoppelte Detektorsonde erkennt. Die Peroxidase geht als Enzym unverändert aus den Reaktionen hervor, da sie mit den Substraten nur kurzlebige Übergangszustände eingeht. Im Zuge der Redoxreaktion, die durch die Peroxidase katalysiert wird, werden der Mediator und das H₂O₂ verändert und es werden Elektronen übertragen, was als elektrischer Strom gemessen werden kann. Der gemessene elektrische Strom ist dann das Maß für die vorhandene Konzentration der zu detektierenden toxischen Alge in der Probe.

Eine Anordnung in Form eines kompletten Sets, das als Frühwarnsystem für den Nachweis toxischer Algen verwendet werden kann, auf Basis einer Sandwichhybridisierung unter Zuhilfenahme einer Helfersonde kann folgende Komponenten enthalten :
- Vorschläge für die Probenaufbereitung
- Beschichtete Einwegbiosensorchips mit den für die nachzuweisenden Algen sequenzspezifischen Fängersonden
- Hybridisierungslösung (Hybridisierungspuffer, Helfersonden und Detektorsonden in Sequenznachbarschaft zu den Fängersonden)
- Hybridisierungsbehälter
- Pipetten
- Lösungen für die Detektion der Sandwichhybridisierung
- Handgerät mit Anzeige und Potentiostateinheit für die elektrochemische Detektion der nachzuweisenden toxischen Algen

### Ausführungsbeispiel zur Sandwichhybridisierung auf einem Einwegbiosensorchip

| | |
|---|---|
| Zu detektierende toxische Alge : | Alexandrium ostenfeldii |
| Fängersonde (Seq.Nr. 19) : | CAA CCC TTC CCA ATA GTC AGG T |
| Detektorsonde : | GAA TCA CCA AGG TTC CAA GCA G |
| Helfersonde a : | TTA ATC TTT GAG ACA AGC ATA TGA AC |
| Helfersonde b : | ACC TGA CTA TTG GGA AGG GTT G |

Im Ausführungsbeispiel wird zur Detektion der toxischen Alge "Alexandrium ostenfeldii" die sequenzspezifische Fängersonde gemäß Sequenz-Nr. 19 verwendet. Die zur Sandwichhybridisierung benötigte Detektorsonde liegt in einem Abstandsbereich von maximal 200 Basen benachbart zur algenspezifischen komplementären RNA- bzw. einzelsträngigen DNA-Sequenz und weist im gewählten Ausführungsbeispiel die oben gezeigte Basensequenz auf. Die Helfersonde, die die Hybridisierung der algenspezifischen, komplementären Algen-RNA oder einzelsträngigen -DNA an die Fängersonde unterstützt, liegt ebenfalls in einem Abstandsbereich von maximal 200 Basen dazu und kann alternativ die oben aufgezeigte Basensequenz a oder b aufweisen. Beide Helfersonden erfüllen die an sie gestellte Aufgabe der Unterstützung der Hybridisierung. An dieser Stelle sei darauf hingewiesen, dass insbesondere bei toxischen Algen der Spezies_"Alexandrium tamarense" (Fängersonde gemäß Sequenz-Nr. 5, 6 oder 18) die Verwendung einer Helfersonde überhaupt erst die Hybridisierung ermöglicht. Diese hochtoxische Algenspezies zeichnet für die Bildung von Neurotoxinen verantwortlich und wurde in der Forschung bereits intensiv untersucht.

### A. Beschichtung der Einwegbiosensoren mit Avidin:

1. Die Einwegbiosensoren (im Folgenden kurz Sensoren) werden mit 50 µl Carbonatpuffer, pH 9,6 angefeuchtet. Der Carbonatpuffer wird mittels einer Vakuumpumpe abgesaugt.
2. Inkubation der Sensoren mit 2 µl Avidin in Carbonatpuffer, pH 9,6 [200 µg/ml] für mindestens 4,5 Stunden bei 4°C (im Kühlschrank).
3. Überschüssiges Avidin wird mit PBS (pH 7,6) abgewaschen, dazu werden die Sensoren mehrmals in eine Petrischale mit PBS (pH 7,6) getaucht. Die Sensoren werden durch Absaugen der Flüssigkeit mit einer Vakuumpumpe getrocknet.
4. Im Anschluss daran werden die Sensoren mit 20 µl 3% Casein in PBS (pH 7,6) 1 Std. bei RT geblockt.
5. Das Casein wird mit PBS (pH 7,6) abgewaschen.
6. Die avidinbeschichteten Sensoren können nach einer Inkubation mit 2% Trehalose in PBS (pH 7,6) mindestens 3 Monate im Kühlschrank gelagert werden. Dazu werden die Sensoren mit 15 µl der Trehalose-Lösung bei 37°C im Wärmeschrank bis zur Verdunstung der Flüssigkeit inkubiert. Vor einer weiteren Verwendung wird die getrocknete Trehalose mit PBS (pH 7,6) abgewaschen.

### B. Immobilisierung der biotinylierten Fängersonde auf den avidinbeschichteten Einwegbiosensoren

1. Die Sensoren werden 30 min mit 2 µl der biotinylierten Fängersonde (DNA-Sonde) [0,1 pmol/µl] in "Beadpuffer" inkubiert.
2. Die Sensoren werden mit 50µl 1x Hybridisierungspuffer beschichtet. Der Hybridisierungspuffer wird im Anschluss direkt abgesaugt.
3. Die DNA-beschichteten Sensoren können nach einer Inkubation mit 2% Trehalose in PBS (pH 7,6) ebenfalls mindestens 3 Monate im Kühlschrank gelagert werden. Dazu werden die Sensoren mit 15 µl der Trehalose-Lsg. bei 37°C im Wärmeschrank bis zur Verdunstung der Flüssigkeit inkubiert. Vor einer weiteren Verwendung wird die getrocknete Trehalose mit PBS (pH 7,6) abgewaschen.

### C. Sandwichhybridisierung von immobilisierter Fängersonde, rRNA der nachzuweisenden Algenspezies und digoxigenin-markierter Detektorsonde

1. Der typische Hybridisierungsansatz umfasst 14µl und enthält 1x Hybridisierungspuffer, 0,25 µg/µl Hering-Sperma-DNA, 0,1 pmol/µl Helfersonde, 0,1 pmol/µl dig-markierte Detektorsonde und 6,5 µl RNA (- 500 ng/µl - 700 ng/µl) der nachzuweisenden Algenspezies, das fehlende Volumen wird mit Wasser aufgefüllt.
2. Der Hybridisierungsansatz wird 4 min bei 94°C erhitzt und danach sofort abgekühlt (Metallblock -20°C). Pro Sensor werden 2 µl des Hybridisierungsansatzes aufgetragen.
3. Es wird 30 min bei 46°C in einer dampfgesättigten feuchten Kammer inkubiert. Die feuchte Kammer sollte zu Beginn der Hybridisierung bereits eine Temperatur von 46°C haben und sollte deshalb gleich zu Beginn des Verfahrens in den Hybridisierungsofen gestellt werden.
4. Nach dem Ablauf der Inkubationszeit bei 46°C werden die Sensoren 5 min bei Raumtemperatur abgekühlt.

### D. Detektion

1. Nach der Hybridisierung werden die Sensoren mit POP-Puffer (pH 6,45) gewaschen.
2. Es folgt eine Inkubation der Sensoren mit 1,5 µl Anti-Dig POD ([7.5 U/ml] in PBSBT) für 30 min bei Raumtemperatur in der dampfgesättigten Inkubationskammer
3. Nach der Inkubation werden die Sensoren mit POP-Puffer (pH 6,45) gewaschen.
4. Danach werden die Sensoren getrocknet und zur Durchführung der Messung in das Handgerät gesteckt. Die Messung erfolgt nach Zugabe von 20 µl POD-Substrat. Die Messung sollte dabei direkt auf den Waschschritt folgen.

### Zu beachten ist:

■ Die einzelnen Schritte sollten zügig nacheinander durchgeführt werden.
■ Die Sensoren werden durch Eintauchen in eine mit dem jeweiligen Waschpuffer gefüllte Petrischale gewaschen.

### Puffer und Lösungen:

### 1. Carbonatpuffer, pH 9,6

| | Konzentration |
|---|---|
| NaHCO₃ | 50 mM |

Der Puffer wird durch einen 0,22µm Membranfilter (Spritzenvorsatzfilter) sterilfiltriert.

### 2. 10 x PBS (0,5 M NaH₂PO₄*H₂O, 1,54 M NaCl, pH 7,4)

| | Konzentration |
|---|---|
| NaH₂PO₄*H₂O | 0,5 M |
| NaCl, pH 7,4 | 1,54 M |

Nach Verdünnung des 10x PBS auf 1x PBS sollte der pH erneut geprüft werden.

### 3. " Beadpuffer"

| | Konzentration |
|---|---|
| NaCl | 0,3 M |
| Tris, pH 7,6 | 0,1 M |

Der Puffer wird nach dem Ansetzen autoklaviert.

### 4. 4x Hybridisierungspuffer

| | Konzentration |
|---|---|
| NaCl | 0,3 M |
| Tris, pH 8,0 | 80 mM |
| SDS | 0,04% |

Vor Gebrauch sollte der Puffer autoklaviert werden.

### 5. 10x POP-Puffer, pH 6,45

| | Konzentration |
|---|---|
| NaH₂PO₄*H₂O | 0,5 M |
| NaCl, pH 6,45 | 1 M |

Die Lösung wird nach dem Ansetzen autoklaviert.

### 6. PBS-BT, pH 7,4

| | Konzentration |
|---|---|
| PBS | 1x |
| BSA | 0.1 % [w/v] |
| TWEEN 20, pH 7,4 | 0,05% [v/v] |

Der Puffer wird durch einen 0,22µm Membranfilter (Spritzenvorsatzfilter) sterilfiltriert

### 7. POD-Substrat

1,1 mg N-Phenyl-1,4-phenylenediamin-hydrochlorid (Mediator ADPA Aminodiphenylamin) werden in 110 µl EtOH gelöst. Diese Lösung wird mit 250 µl 100 mM H₂O₂ (12 µl konz. H₂O₂ + 988 µl H₂O) gemischt und mit 1x POP-Puffer (pH 6,45) auf 25 ml aufgefüllt. Das entspricht 1 mM H₂O₂ und 0,2 mM ADPA.

### Bezugszeichenliste

- **1**: Einwegbiosensorchip
- **2**: Chipträger
- **3**: Sensor
- **4**: Arbeitselektrode
- **5**: Referenzelektrode
- **6**: Hilfselektrode
- **7**: sequenzspezifische Fängersonde
- **8**: Biotin
- **9**: Avidin
- **10**: sequenzspezifische Nukleinsäuresequenz der nachzuweisenden Alge (RNA oder einzelsträngige DNA)
- **11**: Detektorsonde
- **12**: Helfersonde
- **13**: Antigen
- **14**: Enzym
- **15**: Antikörper

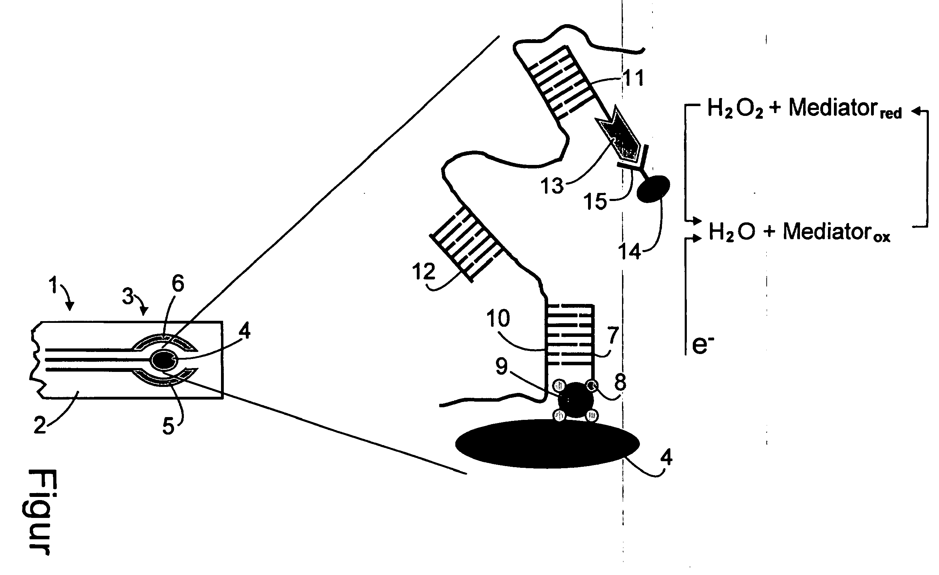

## Patentansprüche

1. Elektrochemisches Nachweisverfahren zur schnellen Vor-Ort-Detektion von toxischen Algen in einer flüssigen Probe unter Verwendung zumindest einer immobilisierten, sequenzspezifischen Fängersonde zur unter Hybridisierungsbedingungen selektiven Hybridisierung einer komplementären, die zu detektierende Alge eindeutig charakterisierenden Nukleinsäuresequenz aus der rRNA der kleinen Untereinheit 18S oder der großen Untereinheit 28S des algenspezifischen Ribosoms, zumindest einer Helfersonde und zumindest einer Detektorsonde, die beide mit einem möglichst geringen Sequenzabstand mit einer maximalen Differenz von 150 bis 200 Basen nahe bei der sequenzspezifischen Fängersonde liegen, mit den hauptsächlichen Verfahrensschritten:
• Aufbereitung der zu untersuchenden flüssigen Probe zur Freisetzung der Gesamt-RNA aller in der Probe enthaltenen Algen,
• Erstellung eines Hybridisierungsansatzes durch Vermischung der freigesetzten Gesamt-RNA mit der Helfersonde und der Detektorsonde, die mit einem Antigen markiert ist, in einem Hybridisierungspuffer zur Hybridisierung an komplementäre Nukleinsäuresequenzen der rRNA des algenspezifischen Ribosoms der in der Probe nachzuweisenden Alge,
• Benetzung der sequenzspezifischen Fängersonde mit dem Hybridisierungsansatz zur Sandwichhybridisierung der die zu detektierenden Alge eindeutig charakterisierenden Nukleinsäuresequenz, wobei die Helfersonde die Sandwichhybridisierung ermöglicht oder verstärkt und die Detektorsonde die zu detektierende Nukleinsäuresequenz markiert,
• Benetzung der sequenzspezifischen Fängersonde nach erfolgter Sandwichhybridisierung mit einem Enzymkonjugat mit einem Antikörper, der spezifisch an das Antigen der Detektorsonde bindet und einem Enzym, das die Detektionsreaktion katalysiert,
• Abspülung von ungebundenem Enzymkonjugat,
• Amperometrische oder .zyklovoltametrische Detektion der bei erfolgter Sandwich-Hybridisierung der zu detektierenden Alge eingetretenen Enzymreaktion bei einer vorgegebenen Messspannung.

2. Elektrochemisches Nachweisverfahren nach Anspruch 1 mit einer Verwendung von zumindest einer sequenzspezifischen Fängersonde gemäß nachfolgender Tabelle :
| **Bezeichng** | **spezifisch für toxische....** | ***)** | **Basensequenz für Fängersonde** | **SEQIDNo** |
|---|---|---|---|---|
| **Klasse** | | | | |
| HETERO01 | Heterokonta | 18S | ACGACTTCACCTTCCTCT | 1 |
| **Gattung** | | | | |
|---|---|---|---|---|
| PRYMGL01 A | Prymnesium | 18S | TGCTCGCCAACGAGGTGT | 2 |
| PRYMGL02 B | Prymnesium | 18S | AAGAAGTGCTCGCCAACG | 3 |
| PSEUDONIT ZSCHIA | Pseudo-nitzschia | 18S | CAGTACAGCGCAATCACT | 4 |
| **Spezies** | | | | |
|---|---|---|---|---|
| ATAM01 | Alexandrium tamarense/ fundyense/catenella species complex | 18S | TTCAAGGCCAAACACCTG | 5 |
| ATNA02 | Alexandrium tamarense (North America) | 28S | AACACTCCCACCAAGCAA | 6 |
| GMIKI01 | Gymnodinium mikimotol species complex | 18S | TTCCGGGCAAGGTCGAAA | 7 |
| PLIMA | Prorocentrum lima | 18S | CCTTCTGCTAAGTCGGGT | 8 |
| PLIMA | Prorocentrum lima | 18S | CTCTAGCATTTCCACGGG | 9 |
| PLIMA | Prorocentrum lima species | 28S | ACACCCCAATTGCCTCGT | 10 |
| Proro Cluster01 | Cluster von Prorocentrum species | 28S | GCAATCAGAACCCATCCT | 11 |
| AOST18S G +15 | Alexandrium ostenfeldii | 18S | CACCAAGGTTCCAAGCAG | 12 |
| AOST18S D | Alexandrium ostenfeldii | 18S | TCCATGTAGCAATCGACC | 13 |
| PSNAUS A-8 | Pseudo-nitzschia australis | 18S | AACGTCGTTCCGCCAAT | 14 |
| PSNMUL A+4 | Pseudo-nitzschia multiseries | 18S | CGCCGCCAAAAGGCATGC | 15 |
| PSNPUNA-12 | Pseudo-nitzschia pungens | 18S | GGGCACCCTCAGTACGAC | 16 |
| AMIN D+1 | Alexandrium minutum | 18S | TCCAGGCAAGGTTGCAAA | 17 |
| Alex Relax | Alexandrium tamarense | 18S | TGCACCTCTGTTGGTRRTACATT | 18 |
| AOST Faenger | Alexandrium ostenfeldii | 18S | (R=A/G) CAACCCTTCCCAATAGTCAGGT | 19 |
| | | | | |
|---|---|---|---|---|
| *) Untereinheit des algenspezifischen Ribosoms, in der die charakterisierende Nukleinsäuresequenz (in rRNA) vorliegt | | | | |

## Claims

1. An electrochemical detection method for fast on-site detection of toxic algae in a liquid sample using at least one immobilized sequence-specific capture probe for hybridization that is selective under hybridization conditions of the complementary nucleic acid sequence uniquely characterizing the algae to be detected from the RNA of the small subunit 18S or the large subunit 28S of the alga-specific ribosome, at least one helper probe and at least one detector probe, both of which are located close to the sequence-specific capture probe with the smallest possible sequence distance, with a maximum difference of 150 to 200 bases, comprising the main process steps:
- Processing the liquid sample to be analyzed to release the total RNA of all algae contained in the sample,
- Creating a hybridization batch by mixing the total RNA thus released with the helper probe and the detector probe which is labeled with an antigen, in a hybridization buffer for hybridization at complementary nucleic acid sequences of the RNA of the alga-specific ribosome of the alga to be detected in the sample,
- Wetting the sequence-specific capture probe with the hybridization batch for sandwich hybridization of the nucleic acid sequence uniquely characterizing the alga to be detected, whereby the helper probe permits or enhances the sandwich hybridization and the detector probe labels the nucleic acid sequence to be detected,
- Wetting the sequence-specific capture probe after successful sandwich hybridization with an enzyme conjugate with an antibody that binds specifically to the antigen of the detector probe and with an enzyme that catalyzes the detection reaction,
- Rinsing off unbound enzyme conjugate,
- Amperometric or cyclovoltametric detection of the enzyme reaction occurring in successful sandwich hybridization of the alga to be detected at a preselected measurement voltage.

2. The electrochemical detection method according to Claim 1 with use of at least one sequence-specific capture probe according to the following table:
| **Name** | **Specific for...** | ***)** | **Base** | **SEQ ID No** |
|---|---|---|---|---|
| **Class** | | | | |
| HETERO01 | Heterokonta | 18S | ACGACTTCACCTTCCTCT | 1 |
| **Genus** | | | | |
|---|---|---|---|---|
| PRYMGL01 A | Prymnesium | 18S | TGCTCGCCAACGAGGTGT | 2 |
| PRYMGL02 B | Prymnesium | 18S | AAGAAGTGCTCGCCAACG | 3 |
| PSEUDONIT ZSCHIA | Pseudo-nitzschia | 18S | CAGTACAGCGCAATCACT | 4 |
| **Species** | | | | |
|---|---|---|---|---|
| ATAM01 | Alexandrium tamarense/ fundyense/catenella species complex | 18S | TTCAAGGCCAAACACCTG | 5 |
| ATNA02 | Alexandrium tamarense (North America) | 28S | AACACTCCCACCAAGCAA | 6 |
| GMIKI01 | Gymnodinium mikimotoi species complex | 18S | TTCCGGGCAAGGTCGAAA | 7 |
| PLIMA | Prorocentrum lima | 18S | CCTTCTGCTAAGTCGGGT | 8 |
| PLIMA | Prorocentrum lima | 18S | CTCTAGCATTTCCACGGG | 9 |
| PLIMA | Prorocentrum lima species | 28S | ACACCCCAATTGCCTCGT | 10 |
| Proro Cluster01 | Cluster von Prorocentrum species | 28S | GCAATCAGAACCCATCCT | 11 |
| AOST18S G +15 | Alexandrium ostenfeldii | 18S | CACCAAGGTTCCAAGCAG | 12 |
| AOST 18S D | Alexandrium ostenfeldii | 18S | TCCATGTAGCAATCGACC | 13 |
| PSNAUS A-8 | Pseudo-nitzschia australis | 18S | AACGTCGTTCCGCCAAT | 14 |
| PSNMUL A+4 | Pseudo-nitzschia multiseries | 18S | CGCCGCCAAAAGGCATGC | 15 |
| PSNPUN A-12 | Pseudo-nitzschia pungens | 18S | GGGCACCCTCAGTACGAC | 16 |
| AMIN D+1 | Alexandrium minutum | 18S | TCCAGGCAAGGTTGCAAA | 17 |
| Alex Relax | Alexandrium tamarense | 18S | TGCACCTCTGTTGGTRRTACATT (R=A/G) | 18 |
| AOST Faenger | Alexandrium ostenfeldii | 18S | CAACCCTTCCCAATAGTCAGGT | 19 |
| | | | | |
|---|---|---|---|---|
| *) Subunit of the alga-specific ribosome in which the characterizing nucleic acid sequence occurs (in RNA). | | | | |

## Revendications

1. Procédé de décèlement électrochimique, pour la détection rapide in situ d'algues toxiques dans un échantillon liquide, en utilisant au moins une sonde de capture immobilisée, à séquence spécifique pour l'hybridation sélective sous conditions d'hybridation d'une séquence d'acide nucléique complémentaire, caractérisant indubitablement l'algue à détecter à partir de l'ARNr de la petite sous-unité 18S ou de la grande sous-unité 28S du ribosome spécifique à l'algue, au moins une sonde auxiliaire et au moins une sonde de détection, situées toutes deux à un intervalle de séquence le plus faible possible, avec une différence maximale de 150 à 200 bases à proximité de la sonde de capture à séquence spécifique, avec les étapes principales de procédé :
• préparation de l'échantillon liquide à analyser pour la libération de l'ARN total de toutes les algues contenues dans l'échantillon,
• élaboration d'une formule d'hybridation par mélange de l'ARNr total libéré avec la sonde auxiliaire et la sonde de détection, qui est marquée avec un antigène, dans un tampon d'hybridation pour l'hybridation sur des séquences complémentaires d'acide nucléique de l'ARNr du ribosome spécifique à l'algue devant être décelée dans l'échantillon,
• imprégnation de la sonde de capture avec la formule d'hybridation pour l'hybridation sandwich de la séquence d'acide nucléique caractérisant indubitablement l'algue à détecter, la sonde auxiliaire permettant ou renforçant l'hybridation sandwich et la sonde de détection marquant la séquence d'acide nucléique à détecter,
• imprégnation de la sonde de capture à séquence spécifique après achèvement de l'hybridation sandwich avec une conjugaison d'enzymes avec un anticorps, qui se lie spécifiquement à l'antigène de la sonde de détection et un enzyme qui catalyse la réaction de détection
• lavage de la conjugaison d'enzymes non liées,
• détection ampérométrique ou cyclo-voltamétrique de la réaction enzymatique ayant eu lieu après achèvement de l'hybridation sandwich à une tension de mesure prédéfinie.

2. Procédé de décèlement électrochimique selon la revendication 1, avec une utilisation d'au moins une sonde de capture à séquence spécifique selon le tableau suivant :
| **Désignation** | **spécifique pour agents toxiques ....** | ***)** | **Séquence de bases pour sonde de capture** | **SEQIDNO** |
|---|---|---|---|---|
| **Classe** | | | | |
| HETERO01 | Heterokonta | 1 18S | ACGACTTCACCTTCCTCT | 1 |
| **Genre** | | | | |
|---|---|---|---|---|
| PRYMGL01A | Prymnesium | 18S | TGCTCGCCAACGAGGTGT | 2 |
| PRYMGL02B | Prymnesium | 18S | AAGAAGTGCTCGCCAACG | 3 |
| PSEUDONIT ZSCHIA | Pseudo-nitzschia | 18S | CAGTACAGCGCAATCACT | 4 |
| **Espèce** | | | | |
|---|---|---|---|---|
| ATAM01 | Alexandrium tamarense/ fundyense/catenella species complex | 18S | TTCAAGGCCAAACACCTG | 5 |
| ATNA02 | Alexandrium tamarense (North America) | 28S | AACACTCCCACCAAGCAA | 6 |
| GMIKI01 | Gymnodinium mikimotoi species complex | 18S | TTCCGGGCAAGGTCGAAA | 7 |
| PLIMA | Prorocentrum lima | 18S | CCTTCTGCTAAGTCGGGT | 8 |
| PLIMA | Prorocentrum lima | 18S | CTCTAGCATTTCCACGGG | 9 |
| PLIMA | Prorocentrum lima species | 28S | ACACCCCAATTGCCTCGT | 10 |
| Proro Cluster01 | Cluster von Prorocentrum species | 28S | GCAATCAGAACCCATCCT | 11 |
| AOST18S G +15 | Alexandrium ostenfeldii | 18S | CACCAAGGTTCCAAGCAG | 12 |
| AOST18S D | Alexandrium ostenfeldii | 18S | TCCATGTAGCAATCGACC | 13 |
| PSNAUS A-8 | Pseudo-nitzschia australis | 18S | AACGTCGTTCCGCCAAT | 14 |
| PSNMUL A+4 | Pseudo-nitzschia multiseries | 18S | CGCCGCCAAAAGGCATGC | 15 |
| PSNPUNA-12 | Pseudo-nitzschia pungens | 18S | GGGCACCCTCAGTACGAC | 16 |
| AMIN D+1 | Alexandrium minutum | 18S | TCCAGGCAAGGTTGCAAA | 17 |
| Alex Relax | Alexandrium tamarense | 18S | TGCACCTCTGTTGGTRRTACATT (R=A/G) | 18 |
| AOST Faenger | Alexandrium ostenfeldii | 18S | CAACCCTTCCCAATAGTCAGGT | 19 |
| | | | | |
|---|---|---|---|---|
| *) Sous-unité du ribosome spécifique aux algues, dans laquelle la séquence d'acide nucléique caractéristique est présente (dans l'ARNr). | | | | |
